(19) Europäisches Patentamt
European Patent Office
Office européen des brevets

(11) **EP 2 426 483 A1**

(12) **EUROPEAN PATENT APPLICATION**

(43) Date of publication:
**07.03.2012 Bulletin 2012/10**

(51) Int Cl.:
***G01N 27/12*** (2006.01)   ***G01N 33/00*** (2006.01)

(21) Application number: **10290469.5**

(22) Date of filing: **02.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicants:
• **University of Southhampton**
  **Southampton, Hampshire SO17 1BJ (GB)**
• **Universite Paul Sabatier Toulouse III**
  **31062 Tolouse Cedex 9 (FR)**
• **Centre National de la Recherche Scientifique CNRS**
  **75016 Paris (FR)**

(72) Inventors:
• **Usgaocar, Ashwin Rajiu**
  **Southampton SO15 2JZ (GB)**
• **de Groot, Cornelis Hendrik**
  **Southampton SO15 2JZ (GB)**
• **Chavagnac, Valerie**
  **Tolouse (FR)**
• **Boulart, Cedric**
  **31400 Tolouse (FR)**

(74) Representative: **Haines, Miles John L.S.**
  **D Young & Co LLP**
  **120 Holborn**
  **London EC1N 2DY (GB)**

(54) **Hydrogen Sensors**

(57)   A hydrogen sensor formed using electrodeposition to deposit thin films of hydrogen-catalytic metal. A substrate terminating in a semiconductor layer is oxidised and then photolithographically patterned to create windows exposing areas of the semiconductor. Schottky contacts are then formed by electrodepositing Pd, PdNi or Pt onto the exposed areas of the semiconductor. A device structure with back-to-back Schottky junctions is particularly well suited to fabrication by electrodeposition. Electrodeposited PdNi alloys form Schottky barriers with extremely low reverse bias currents, and the back-to-back configuration ensures that in use current flow through the device is kept extremely low.

Fig. 3A

EP 2 426 483 A1

**EP 2 426 483 A1**

**Description**

**BACKGROUND OF THE INVENTION**

The invention relates to hydrogen sensors and to methods of manufacturing hydrogen sensors.

A known class of hydrogen sensors exploits the catalytic properties of palladium, platinum and related alloys. These sensors can be sub-classified into various types.

A first type is based on a metal-insulator-semiconductor (MIS) field effect transistor (FET). This type includes metal-oxide-semiconductor (MOS) FETs. MISFET or MOSFET sensors have an FET structure with a layer of hyrdogen-catalysing metal forming the gate. Dissociation and diffusion of hydrogen through the gate creates a dipole layer of charges and changes the threshold voltage of the device, which can be used to sense hydrogen pressure. While these devices exhibit high sensitivity, their power consumption is relatively high, because a conductive path always exists even in the absence of hydrogen.

Lundström et al [1] disclose a hydrogen sensor of the first type. The structure is a MOSFET of silicon, silicon dioxide and Pd gate.

Hughes & Schubert [2] disclose hydrogen sensors of the first type. Fabrication is by electron beam evaporation. PdNi alloy is used as the catalyst metal. It is also mentioned that not only Pd but also PdAg alloy are known as catalyst metals in the prior art. In the PdNi alloy, it is said that "for Ni content up to 26%, the diffusion of H is not severely impeded." Results are presented for 8% and 15% Ni, and samples in the range 8-20% are said to have been fabricated and tested. In a subsequent publication by the same authors, Hughes, Schubert & Buss [3], there is disclosed similar structures to those just described [2] with specific examples at 3, 5, 8, 15 atom% Ni in a PdNi alloy. In addition, an upper limit of Ni concentration of 56 atom% is given, above which the PdNi alloy does not have its catalytic properties in respect of hydrogen. A concentration of around 10 atom% Ni is also said to be a good trade-off between the relevant factors. Hydrogen absorption by palladium is known to induce an irreversible phase change from a so-called alpha phase to a so-called beta phase, which limits the performance of the device. Alloying palladium with nickel is shown to delay the onset of this irreversible phase change [2], increasing the range of hydrogen concentrations over which the sensor can function and also allowing the sensor to recover and be used for multiple measurements.

US 6,935,158 [4] discloses an example sensor of the first type. The MIS structure is based on a metal of Pd an insulator of AIN and a semiconductor of Si or SiC. Fabrication is by molecular beam epitaxy (MBE). The two known problems of palladium are also mentioned. First, the hydrogen-induced alpha to beta phase transition. Second, the embrittlement of palladium that results from cycling the sensor in use through the repeated absorption then desorption of hydrogen which has the effect of repeated expansion and contraction of the palladium. It is also stated that to overcome these known problems it has been suggested to alloy Pd with Ni to form a PdNi alloy or Pd with Group V elements. However, such approaches are said to have temperature range limitations which mean that high temperature operation is not possible. The following materials are said to be suitable hydrogen sensing materials: "Pd, Pt, Rh, and alloys thereof".

A second type of hydrogen sensor is based on deposition of a thin film of Pd or related metal on a semiconductor substrate and sensing the change in film resistivity in the presence of hydrogen that has diffused through the Pd.

US 5,367,283 [6] discloses an example sensor of the second type. The sensor operates by measuring the difference in resistance between two similar Pd films, one of which is exposed to hydrogen, and the other is not. The Pd films are deposited on a substrate, such as alumina, which plays no role in the sensing circuit. First and second metallization layers are deposited on the substrate before depositing the Pd films in order to ensure adhesion of the Pd films. The text refers to Pd or Pd alloy films, but no mention is made of any specific material to alloy with Pd.

Hughes & Schubert [2] already mentioned above also disclose hydrogen sensors of the second type, with the same compositions as discussed above for the first type. Hughes, Schubert & Buss [3] disclose similar structures to those in Hughes & Schubert [2] with specific examples at 3, 5, 8, 15 atom% Ni in a PdNi alloy.

A third type of hydrogen sensor is based on forming a metal-semiconductor Schottky junction between palladium or platinum and a suitable semiconductor. A semiconductor substrate typically with a suitable n-type epitaxial layer thereon has a localised film of Pd deposited on the semiconductor which forms a Schottky barrier at the metal-semiconductor interface. The presence of hydrogen in the Pd has the effect of reducing the Schottky barrier height, since hydrided Pd has a lower work function than pure Pd. This lowering of the work function is detectable through the resulting change in the current-voltage (I-V) characteristic of the junction, so the I-V characteristic allows the hydrogen concentration in the Pd, and hence the atmosphere, to be deduced. Such Schottky barrier devices measure the current in a path through the Schottky barrier and then out of the semiconductor via an Ohmic contact arranged adjacent to the Pd contact.

US 6,293,137 [7] discloses an example sensor of the third type. InP is used as the substrate material with an n-type layer of InP thereon, with Pd as the hydrogen-disassociating metal and AuGe alloy as the Ohmic contact material. The generalisations within the patent [7] are that the semiconductor may be III-V group and that either pure Pd or a Pd alloy may be used, although no specific alternative materials are mentioned either for the semiconductor or for the element or elements to alloy with Pd. Fabrication is by metallo-organic vapour phase deposition (MOCVD).

US 6,160,278 [8] discloses an example sensor of the third type which has essentially the same structure as that of US 6,293,137 [7], but uses different materials and fabrication. Fabrication is by molecular beam epitaxy (MBE). The materials combination is GaAs substrate with n-type GaAs epitaxial layer, Pd Schottky contact and AuGe Ohmic contact. The document contains no generalisations.

Huang et al [5] disclose an example sensor of the third type. The materials combination is a sapphire ($Al_2O_3$) substrate with an n-type GaN epitaxial layer, Pd Schottky contact and TiAl Ohmic contact. Fabrication is by MOCVD.

Schalwig et al [9] disclose an example sensor of the third type. The materials combination is a sapphire ($Al_2O_3$) substrate with an n-type GaN epitaxial layer, Pt Schottky contact and AuTiAl Ohmic contact. Fabrication is by MBE.

Weichsel et al [10] disclose an example sensor of the third type. The materials combination is $n^+$ GaAs substrate on which is deposited a ZnO layer. A Pd Schottky contact is deposited on the ZnO. An Indium Ohmic contact is made to the bottom of the substrate. Fabrication is by MOCVD. This article also refers to work of others on a similar structure in which Pt is used instead of Pd.

Hydrogen sensors of the third type tend to draw significant current when idle which may be in the micro- or even milli-ampere range. The device therefore consumes significant power, which is a challenge for battery-operated devices.

A fourth type of hydrogen sensor exploits the properties of a Schottky junction similar to the third type. However, in the fourth type two adjacent Schottky contacts are formed on the semiconductor, instead of a Schottky contact and an Ohmic contact. The sensor of the fourth type has a current path between two adjacent Schottky contacts formed on the semiconductor so that the equivalent circuit of the sensor device is one of two back-to-back diodes interconnected by a conductive path through the semiconductor.

Wang et al [11] disclose a hydrogen sensor of the fourth type in which a GaN/AlGaN heterostructure is grown by MOCVD on a sapphire ($Al_2O_3$) substrate, and the metal Schottky-barrier forming contacts are Pt.

With hydrogen sensors of these types, fabrication is conventionally carried out using a combination of vapour deposition and photolithography. The vapour deposition methods used typically involve either vacuum systems (e.g. MBE) or complex gas handling (e.g. MOCVD) or both. A consequence of this is that the palladium or other metal is initially deposited over a larger area, and then the excess removed by a lift off process, i.e. removal of the photoresist with overlying metal. These methods result in significant waste of metal through the lift-off process. This waste is significant in view of the high cost of palladium and platinum.

## SUMMARY OF THE INVENTION

The invention provides in one aspect a method of manufacturing a hydrogen sensor comprising: providing a substrate terminating in a semiconductor layer; forming an insulating layer on the semiconductor layer; selectively removing areas of the insulating layer to expose corresponding areas of the underlying semiconductor layer; and electrodepositing onto the exposed areas of the semiconductor layer a contact made of a metal that disassociates hydrogen molecules (e.g. Pd, PdNi, Pt) and that forms a Schottky barrier with the semiconductor layer.

In the electrodeposition process, the metal contact is deposited by passing a current through a metal ion solution with the substrate as the cathode. The metal ions collect electrons at the cathode, are reduced and deposited on the substrate to form the metal contact. The reduction and the subsequent metal deposition occurs only at areas of the cathode which are exposed to the electrolyte and the metal is deposited only in the required areas with no wastage. This is a significant improvement over use of conventional deposition which will need to be combined with a lift-off process. Electrodeposition also does not require any special vacuum conditions and can be performed on a table top at room temperature or with localised heating, thereby considerably simplifying Schottky barrier fabrication.

If the metal is an alloy, such as PdNi, good control can be achieved over the composition of the alloy by using different combinations of deposition potential and concentrations of electrolytes.

The electrodeposition process is compatible with the use of silicon, which provides compatibility with conventional integrated circuit design and low fabrication costs.

The electrodeposition preferably takes place in two stages, comprising a first stage with relatively high potential in order to promote nucleation of metal islands followed by a second stage with reduced potential to allow stable depositions to form across the desired area.

The electrodeposition method is particularly simple in the case that the sensor device is of the fourth type mentioned further above, i.e. is based on two adjacent Schottky contacts formed on the semiconductor layer which provide a sensing circuit of two back-to-back diodes interconnected by a current path through the semiconductor layer. This is because the electrodeposition provides both contacts to the semiconductor in a single process step. As well as being ideally suited to manufacture by electrodeposition, there is a device performance advantage of the back-to-back Schottky diode configuration (fourth type). The current consumption of a hydrogen sensing device based on back-to-back Schottky diodes can be limited to extremely low values, because one of the diodes is always in reverse bias. This ensures that the device always consumes low power, even when exposed to voltage spikes which can potentially drain large currents from a single Schottky barrier device (third type) by forcing it into forward bias. By suitable sizing of the area of the Schottky contact, it is possible to provide a sensor device which maintains its current in the nanoampere range for all hydrogen concentrations and pressures, thereby ensuring low power consumption over a wide range of conditions. Low power consumption is significant, since for many applications hydrogen sensors will have constrained power supplies, such as batteries.

The electrodeposition method is also applicable in the case that the sensor device is of the third type mentioned further above, i.e. is based on adjacent pairs of Schottky and Ohmic contacts, wherein the electrodeposition method is used to deposit the Schottky contact, and optionally also the Ohmic contact. The order in which the contacts are fabricated can be chosen as desired, e.g. the Ohmic contact may be fabricated before or after the Schottky contact. Moreover, in one embodiment, a sensor device of the third type is fabricated using the same electrodeposition step for both Schottky and Ohmic contacts, wherein the Ohmic contact is formed by selective doping of the region of semiconductor underlying the metal, e.g. by ion implantation, so that the same metal that forms the Schottky contact also forms the Ohmic contact by virtue of the selective doping.

In a preferred embodiment, the Schottky contact (third type) or Schottky contacts (fourth type) are made of a hydrogen-disassociating PdNi alloy. According to the literature [3], the hydrogen disassociating property of PdNi alloy reduces gradually as the Ni content increases, and eventually a Ni content is reached at which the property is substantially entirely lost. According to the literature [3] an PdNi alloy with 56atom% Ni has no hydrogen disassociation property. However, contrary to this prior art finding, the present inventors have found hydrogen gas response in a PdNi sample with 67atom% Ni. A preferred embodiment of the invention therefore relate to PdNi alloy having a Ni content in a range with a lower bound of 3, 5, 8 or 10 atom% and an upper bound of 12, 15, 20, 25, 30, 35, 40, 45, 50, 55, 60 or 65 atom% with any combination of the lower and upper bound values being contemplated. Generally speaking higher concentrations of Ni and lower concentrations of Pd result in less sensitivity to hydrogen, but better protection against phase change and embrittlement, so the choice of Ni content is a trade-off which should take account of the expected operating environment of the sensor including factors such as the desired operating temperature range and expected range of possible hydrogen concentrations.

The hydrogen catalysing material for the Schottky contact or contacts may be Pd, a Pd alloy, most especially PdNi, Pt or a Pt alloy.

The semiconductor layer may be Si or a Si alloy such as SiGe or SiC, or Ge. The semiconductor layer may be GaAs or a ternary or quaternary compound including Ga and As (e.g. GaAsP, GaAsInP), InP or a ternary or quaternary compound including In and P, or GaN or a ternary or quaternary compound including Ga and N (e.g. AlGaN). The semiconductor may be doped to a suitable resistivity level, e.g. between 0.1 and 10 $\Omega$cm or 0.01 and 100 $\Omega$cm.

Ohmic contacts, if provided, may be made of any suitable materials known in the art to form an Ohmic contact with the selected semiconductor material. A relatively wide variety of options are known from the literature and form part of the general knowledge of a person skilled in the art. Known examples include: AuSb alloy for n-type Si; AlIn alloy for GaN; In or AuSn for InP; or AuGe or AuSn for n-type GaAs.

The insulator may be $SiO_2$ or $Si_3N_4$, or any other suitable insulator for the particular semiconductor being used.

In summary of a first embodiment, a hydrogen sensing device has been described which comprises: a semiconductor layer; first and second metal contacts made of a PdNi alloy which has the property of disassociating hydrogen molecules and arranged adjacent to each other on the semiconductor layer, the first and second metal contacts having respective first and second interface surfaces that form first and second Schottky barriers with the semiconductor layer, and respective first and second sensing surfaces exposed to the atmosphere; and a sensing circuit operable to measure current that flows in a path from the first metal contact into the semiconductor and from the semiconductor to the second metal contact, the first and second metal contacts thereby forming back-to-back Schottky diodes in the sensing circuit, said current being sensitive to molecular hydrogen present at the sensing surfaces.

In summary of a second embodiment and a third embodiment, a hydrogen sensing device has been described which comprises: a semiconductor layer; first and second metal contacts arranged adjacent to each other on the semiconductor layer, wherein the first metal contact is made of PdNi alloy which has the property of disassociating hydrogen molecules and forms a Schottky barrier with the semiconductor layer, and has a sensing surface exposed to the atmosphere; the second metal contact is made of a material that forms an Ohmic contact with the semiconductor layer; and a sensing circuit operable to measure current that flows in a path from the first metal contact into the semiconductor via the Schottky barrier and from the semiconductor to the second metal contact via the Ohmic contact, said current being sensitive to molecular hydrogen present at the sensing surface.

In the second embodiment, the second metal is different from the first metal and forms an Ohmic contact with the semiconductor. In the third embodiment, the second metal is the same as the first metal and forms an Ohmic contact with the semiconductor by virtue of local doping of the semiconductor at its interface with the second metal.

The first and second metal contacts have a thickness of preferably between 10-1000nm, more preferably 30-300 nm, still more preferably 50-200 nm.

First and second contact pad electrodes may additionally be provided which in one part are in Ohmic contact with the first and second metal contacts and in another part with first and second probe leads to a measurement unit. The first and second contact pad electrodes may be made of any suitable materials known in the art to form an Ohmic contact with the metal used for the first and second contacts and with the metal used for the first and second probe leads. Example materials are Al or Au, or alloys of either Al or Au. The first and second contact pads preferably provide a sufficiently large area for ease of connection to the probe leads. The first and second contact pads are also preferably arranged so as to leave a substantial area portion of the first and second metal contacts free to provide the first and second sensing surfaces. A substantial area portion may be at least 15, 30, 40, 50, 60, 70 or 80% of the total area.

In summary, an electrodeposition method is used to fabricate the contacts made from the hydrogen disassociating material. Preferred embodiments alloy palladium with nickel in order to provide improved properties compared with pure palladium for a Schottky contact-based device (third type or fourth type). Moreover, a device structure with back-to-back Schottky junctions made of the same material is particularly well suited to a simple fabrication process using electrodeposition.

The most preferred combination is a PdNi alloy back-to-back Schottky device on a Si substrate and Si epitaxial layer with $SiO_2$ as the insulator in which the PdNi is electrodeposited. Electrodeposited PdNi alloys were found to form Schottky barriers with extremely low reverse bias currents, and the back-to-back configuration ensures that only the low reverse bias current will be flowing through the device in use.

## DEFINITIONS

**metal:** a material having metallic conductivity, thereby including not only metals, but also metallic materials such as silicides or alloys of a metal and a non-metallic material such as AuGe alloy.

**semiconductor:** a semiconducting material, i.e. one with a non-zero band gap

**Schottky contact:** a rectifying junction formed at a metal-semiconductor interface in which a potential barrier exists which inhibits majority carrier transport from the semiconductor to the metal, e.g. electron transport in the case of an n-type semiconductor. The size of the potential barrier scales - among other things - with the work function of the metal, and is also varied by applying a voltage across the junction (biasing).

**Ohmic contact:** a contact formed at the interface between two materials in which charge carriers can pass freely between materials in response to a voltage difference being applied. In the case of a wide band gap semiconductor to metal interface an Ohmic contact is usually produced through high doping of the semiconductor, since metals are generally not available with a sufficiently low work function. For example, Au-Sb may be used for Si or Ge, or Au-Ge or Au-Sn for GaAs. It is also possible to assist formation of an Ohmic contact between a metal and a semiconductor by heavy doping of the interface region of the semiconductor, in particular degenerate doping of the semiconductor.

## BRIEF DESCRIPTION OF THE DRAWINGS

The invention is now described by way of example only with reference to the following drawings.

Figure 1 shows schematic cross-sections at various stages during fabrication of a device structure according to a first embodiment.

Figure 2 is a schematic drawing of electrodeposition equipment used during fabrication.

Figure 3A is a schematic cross-section of the device of the first embodiment with its hydrogen sensing circuit features indicated.

Figure 3B is a schematic plan view of the device of Figure 3A.

Figure 4 is a graph showing the Ni atomic fraction in the PdNi alloy films deposited by electrodeposition at different applied potentials between the cathode and anode.

Figure 5 is a plan view of a test chip.

Figure 6A is a graph showing the dependence of device current on the exposed length of a $Pd_{0.71}Ni_{0.29}$ sensing element.

Figure 6B is a graph showing the dependence of current with hydrogen gas pressures for three example devices.

Figure 7 shows schematic cross-sections at various stages during fabrication of a device structure according to a second embodiment.

Figure 8 shows schematic cross-sections at various stages during fabrication of a device structure according to a third embodiment.

**DETAILED DESCRIPTION**

In the following detailed description, references to % content in alloys, in particular % Ni, refer to atomic % in the alloy, not weight %.

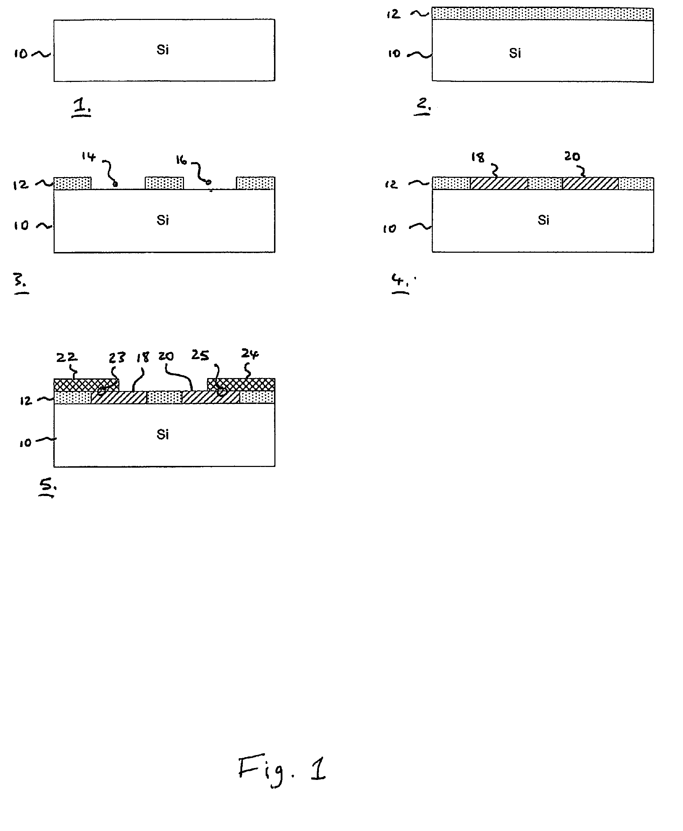

Figure 1 shows schematic cross-sections at five stages during fabrication of a device structure according to a first embodiment. The embodiment will be described with reference to a specific example, but the structure is generic to a range of materials combinations and other variations as specified elsewhere in this document.

In Stage 1, a semiconductor surface layer 10 is provided which may be the substrate itself as illustrated, or may be a separate semiconductor layer arranged on a substrate. In this example, a silicon substrate is provided in the form of an n-type 2-inch wafer of resistivity 0.5-1.5 $\Omega$cm and crystallographic orientation <100>, which provides the semiconductor surface layer. In other words, there is no epitaxy involved. The wafer was cleaned by a 2 minute dip in fuming nitric acid (FNA) followed by rinsing with deionised (DI) water.

In Stage 2, an oxide layer 12 is grown on the substrate to act as an insulating layer. In the case of a Si surface, a $SiO_2$ layer can be grown thermally. In the specific example, a 50nm $SiO_2$ has been grown on the wafer.

In Stage 3, the oxide layer 12 is patterned using photolithography and the exposed oxide is etched by a 20:1 hydrofluoric acid (HF) etch to form adjacent windows 14, 16 through the oxide to the semiconductor surface.

In Stage 4, a metal which has hydrogen catalysing properties is deposited using an electrodeposition process onto the semiconductor surface in the areas defined by the windows 14, 16 to form first and second metal contacts 18 and 20. At the respective interfaces 19, 21 between the metal contacts 18, 20 and the semiconductor 10, respective Schottky barriers are formed. The metal contacts 18, 20 formed of the hydrogen catalysing metal are referred to in the following as Schottky contacts for convenience. In this example, the hydrogen catalysing metal is a nickel-palladium alloy. Other metals that disassociate hydrogen molecules can be used, such as pure Pd, Pt or a Pt alloy. For the electrodeposition, the entire wafer is immersed in an electrochemical bath containing a salt of the metal and the metal is electrodeposited on the exposed silicon surface to form Schottky diodes. The electrodeposition process is described separately below.

In Stage 5, a second photolithography step is carried out to define patterns of a further metal. The purpose of the further metal is to provide contact pad electrodes to facilitate attachment of probe leads or wires. As illustrated, contact pads 22, 24 are formed to contact the respective Schottky contacts 18, 20, while at the same time leaving a substantial area portion of the upper surface of the Schottky contacts 18, 20 exposed so that in use they can adsorb hydrogen. The further metal is selected so as to form an Ohmic contact at the respective interfaces 23, 25 between the contact pads 22, 24 and the hydrogen catalysing metal contacts 18, 20. The further metal is aluminium in this example and is deposited by evaporation and lift-off. The thickness of the aluminium contact pads is approximately 100 nm in this example.

While the process has been described with reference to concrete example materials, it will be appreciated that other materials can be used for the semiconductor, insulator, first metallisation and second metallisation, as disclosed elsewhere in this document. The basic device structure as illustrated in Figure 1 will remain substantially the same independent of the materials selections. Moreover, the second metallisation is provided for practicality, and is not essential to the operational principles of the device. Additional layers may be provided in some embodiments. For example, between the substrate and the insulating layer, there may be epitaxial layers of semiconductor. Indeed, the substrate may not be a semiconductor, for example it may be sapphire, and the semiconductor is provided as a layer on the substrate.

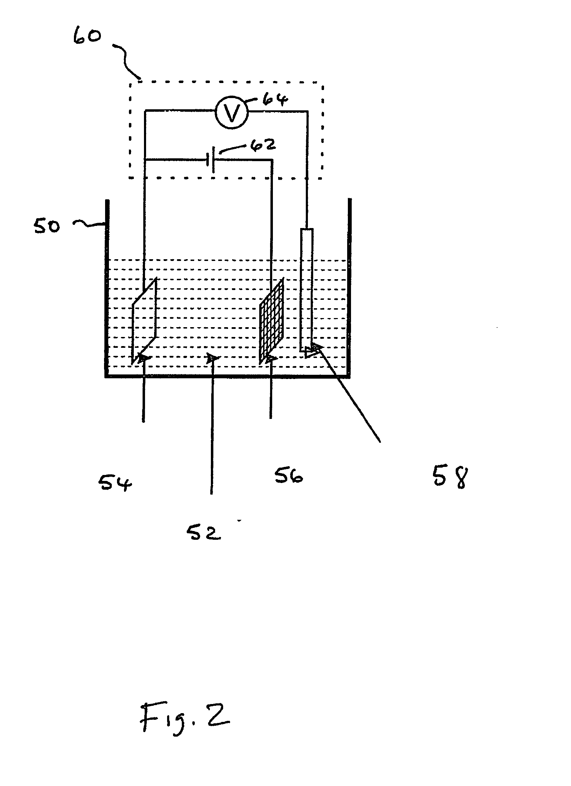

Figure 2 is a schematic drawing of electrodeposition equipment used during fabrication. Electrodeposition is a process for depositing thin metal films by passing an electrical current through a solution of the metal salt (electrochemical bath) .

An electrochemical bath container 50 contains a salt solution 52 which is the electrolyte. There are three electrodes 54, 56, 58 immersed in the bath. The first electrode 54 forms the cathode and is made of the target substrate 10. The second electrode 56 is a platinum mesh used as the anode. The third electrode 58 is a reference electrode which is used to ensure that the potential applied between the anode and cathode remains the same independent of changes in concentration of the salt solution that take place during the electrodeposition, or changes that occur during formation of the metal film on the cathode. The process is controlled by a control unit 60 comprising a voltage source 62 and a voltmeter 64 with appropriate control circuitry (not shown).

When a potential difference is applied between anode and cathode, an electric field is set up in the solution that causes the dissolved metal ions (positively charged) to migrate to the cathode. Once the ions reach the cathode, they collect electrons from an exposed and conducting part of the cathode, get reduced and are deposited on the cathode surface. Subsequent growth can occur by ions gaining electrons from the cathode surface or any part of the deposited film on the cathode.

Binary alloy depositions are accomplished by using an electrochemical bath of two metal salts, whose concentrations are adjusted so as to obtain a potential range where both metals will be deposited [12]. The electrodeposition of PdNi alloys was done by using the recipe for an electrochemical bath from Ref. [13]. Three baths (Ni-only, Pd-only and PdNi) were prepared as shown in Table. 1.

Table 1: Concentrations of components in the three solutions prepared for each combination of Ni and Pd salts.

| Components | Ni bath(g/L) | Pd bath(g/L) | PdNi bath(g/L) |
|---|---|---|---|
| $NiSO_4:6H_2O$ | 44.40 | 0 | 44.40 |
| $Pd(en)Cl_2$ | 0 | 11.1 | 11.1 |
| $(NH_4)_2SO_4$ | 16.70 | 16.70 | 16.70 |
| $NH_3(35\%)$ | 45ml/L | 45ml/L | 45ml/L |

Cyclic voltammetry (CV) curves were measured for each of the three baths by cycling the deposition potential from 0V to -3V and back to study the dependence of the current in each bath as a function of the deposition potential.

The preferred practice is to apply a short and high voltage pulse to the system to promote nucleation of metal particles on the cathode surface. Once this nucleation is complete, a lower voltage is applied that causes the film to grow primarily by increasing the size of islands already deposited on the surface.

For the example of a PdNi alloy on Si, a high-potential pre-treatment pulse of around -2.5 V was applied with the silicon chip as the cathode. The PdNi alloy was electrodeposited using a lower potential such that the deposition current density was maintained in the 3-5mA/cm$^2$ range - which typically involves potentials in the range -1.2 V to -1.6 V. The pre-treatment pulse serves to promote nucleation on the silicon surface and improves film adhesion by providing a large number of anchor points, nucleation centres or islands, on the silicon for subsequent accumulation of the metal. The subsequent deposition is carried out at a lower potential so that the subsequent deposition is dominated by depositing metal onto previously created islands, rather than formation of new islands. The current density of 3-5mA/cm$^2$ was chosen because densities outside this range were found to adversely affect the film quality - yielding discontinuous and brittle films.

For this PdNi bath, a short pulse of relatively high potential of -2.5 $\pm$ 0.5 V is applied, preferably -2.5 $\pm$ 0.2 V, followed by a lower potential selected to provide a current density of between 3-6 mA/cm$^2$ and preferably between 3-4 mA/cm$^2$ which is typically in the range -1.2 V to -1.8 V. Other baths may have different optimum ranges of voltages for the pulse step or the lower potential step.

A mechanism to control the composition of the alloy was devised by assuming that the individual chemical reactions involved in the PdNi bath are independent. The nominal value for the film composition can be calculated using the formula

$$^{Ni}Nom = \frac{I_{Ni}(V_d)}{I_{Ni}(V_d) + I_{Pd}(V_d)} \qquad (1)$$

Where $I_{Ni}(V_d)$ and $I_{Pd}(V_d)$ are the currents measured in the Ni-only and Pd-only baths at a deposition potential $V_d$. The nominal value was compared to the film composition as measured using energy dispersive X-Ray (EDX) spectroscopy.

To deposit PdNi alloy as the hydrogen-disassociating metal in a specific example, the electroplating solution is as specified in Table 1 above, namely it is made of Ni-Sulphate and Pd-ethylenediamine dichloride, ammonium sulphate, ammonia and water, preferably 44.40 g/L Ni-Sulphate, 11.1 g/L Pd-ethylenediamine dichloride, 16.70 g/L ammonium sulphate, 45ml/L of 35% ammonia and remainder water.

Pure Pd films can be deposited in essentially the same way using the Ni-free bath specified in the middle column of Table 1. Another example of a 0% Ni (pure Pd) sample that has been fabricated used the following bath: 38.85 g/L $Pd(en)Cl_2$, 16.70 g/L ammonium sulphate, 45ml/L of 35% ammonia and remainder water. The deposition potential was -1.05V with one pulse of -2.5V which was applied for 0.5s. As a general comment, it should be noted that an extremely wide range of combinations of pulse potential and deposition potential will give a pure Pd film with this bath, because there is no NiSO$_4$ in the solution.

For pure Pt films, Pt electrodeposition may can be performed with the following components [15]:

■ 4.54 g/L Na$_2$PtCl$_6$ - Hexachloroplatinate
■ 0.1 mol/L H$_2$SO$_4$ - Sulphuric acid

Deionised water can be used as the solvent. An alternative is to use K$_2$PtCl$_6$ instead of Hexachloroplatinate.

As described further above, electrodeposition takes place after the oxide layer has been selectively removed by etching to form windows through to the semiconductor. It is noted that, if the process is carried out in normal atmospheric conditions, i.e. air at room temperature, some re-oxidisation of the windows will occur in the time interval between completing the etch and immersing the wafer in the salt solution. However, the re-oxidisation layer will be very thin of the order of 1 nm or less, for example less than 3, 2 or 1 nm, and has no significant deleterious effect on the electrical properties of the metal-semiconductor interface. If it is desired that re-oxidisation of the Si is to be suppressed, then a nitrogen or helium atmosphere could be used to handle the wafer.

In fact, the presence of a very thin oxide layer, or other insulator layer, may be desired, since its presence will limit diffusion between the metal and the semiconductor. It may also serve to de-pin the Fermi level so that the change in metal work function causes a corresponding change in the Schottky barrier height. Undesired pinning may result from metal induced gap states. An oxide layer may therefore be grown in a controlled way in some embodiments, or another kind of insulator such as silicon nitride deposited to perform the same role. The possible presence of a thin re-oxidisation layer, or other insulating layer, is therefore ignored elsewhere in this document.

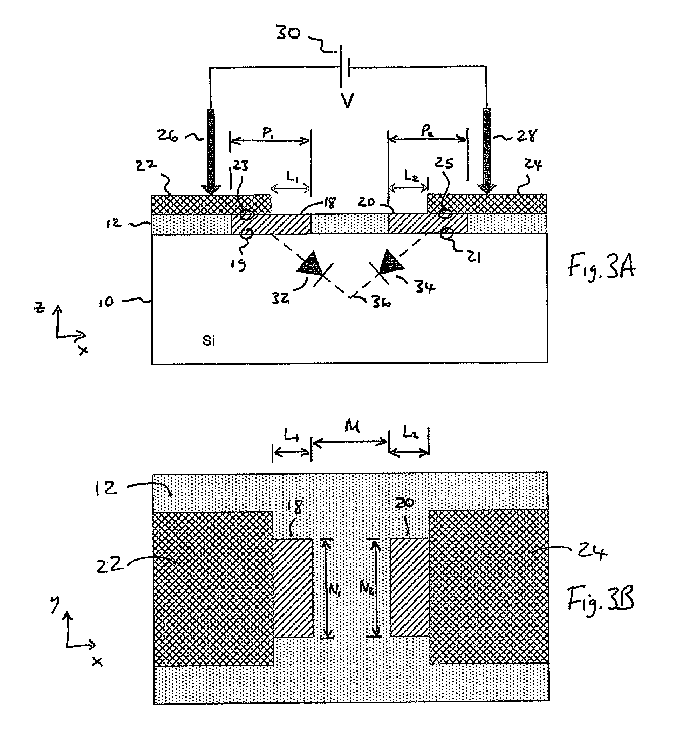

Figure 3A is a schematic cross-section of the device of the first embodiment. The structure is the same as illustrated in the last drawing of Figure 1, but additionally shows features of the hydrogen sensing circuit. Figure 3B is a schematic plan view of the device of Figure 3A.

Probe leads 26, 28 are in Ohmic contact with respective contact pads 22, 24 and lead to a voltage source 30 which is operable to vary the potential applied across the device. The two metal-semiconductor interfaces 19, 21 form Schottky barriers as schematically illustrated by respective diodes 32, 34. (For ease of illustration, the diodes are not drawn at the precise location of the metal-semiconductor interface.) The diodes are sketched as being connected back-to-back by a lead or wire 36 which represents the conductive path between the two Schottky barriers provided by the n-type semiconductor 10.

Referring to both Figure 3A and Figure 3B, the Schottky contact 18 has dimensions P$_1$ x N$_1$ and the Schottky contact 20 has dimensions P$_2$ x N$_2$ in the x and y directions respectively. In the illustrated example P$_1$=P$_2$=P and N$_1$=N$_2$=N. The area portion of the upper surface of the Schottky contact 18 that is exposed for absorption of hydrogen has dimensions L$_1$ x N$_1$ in the x and y directions respectively. The corresponding area of the Schottky contact 20 is L$_2$ x N$_2$. In the illustrated example L$_1$=L$_2$=L. Moreover, the Schottky contacts 18, 20 are separated from each other by a distance M. In the test device the parameter values were M = 10 - 100 $\mu$m, P = 70 $\mu$m and N = 20 $\mu$m. In the test device the exposed dimension in the x direction L is different for the different multiple Schottky contact pairs and has values in the range 10-55 $\mu$m so that the exposed area is varied between approximately 15% and 80% of the total Schottky contact area.

In summary, Figures 3A and 3B show a sensor device structure which has an electrodeposited nickel-palladium alloy in direct contact with the underlying silicon and the evaporated aluminium contact pads which are separated from the silicon by an insulating oxide layer. There is an area of overlap between the nickel-palladium alloy and the aluminium which forms an Ohmic contact, the remainder of the upper nickel-palladium film surface being exposed to the atmosphere so it can absorb hydrogen in use. The exposed length is a factor in deciding the rate of increase of the current before a steady state is achieved.

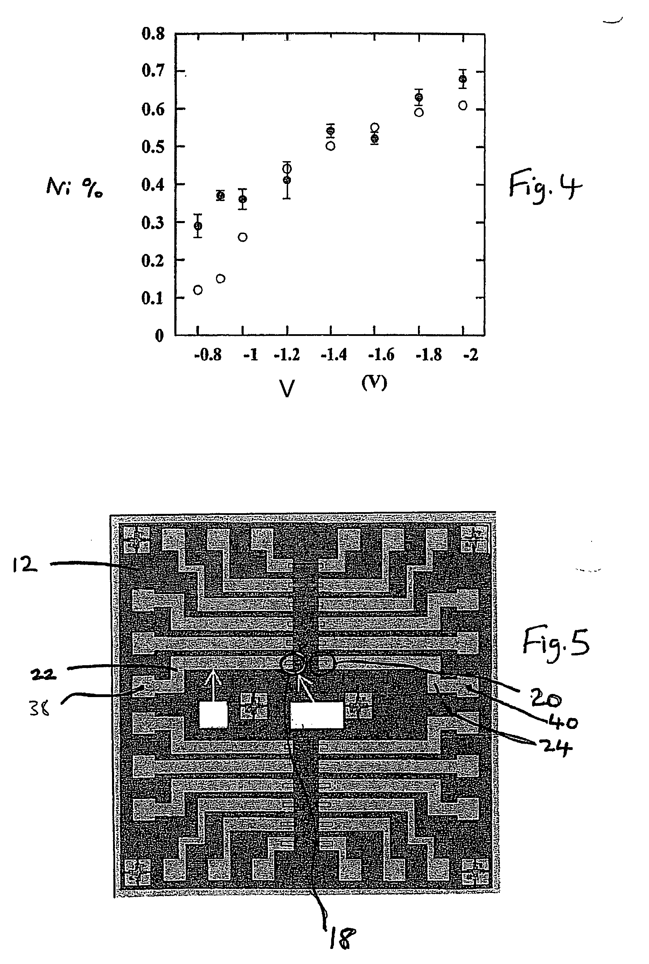

Figure 4 is a graph showing the Ni atomic fraction % in the PdNi alloy films deposited at different potentials V from a 44.4g/L NiSO$_4$:6H$_2$O and 11.1 g/L Pd(en)Cl$_2$ bath. Nominal values are shown with hollow circles and EDX measured values with filled circles. Close agreement between the nominal and experimental values of the atomic Ni fraction is observed showing that the process has the ability to accurately deposit an alloy of desired composition. The accuracy of the prediction is higher for potentials more negative than -1.2V. The plot illustrates samples that have been fabricated over a wide range of Ni concentrations in the range from 29atom% to 68atom% which were all obtained from a single electrochemical bath by varying the deposition potentials.

In general, it is possible to obtain lower Ni concentrations by either lowering the concentration of NiSO$_4$, or by using a less negative electrodeposition potential, or by not applying the initial higher potential nucleating pulse, or a combination of these three factors.

For example, a sample has been fabricated with 13% Ni from a bath with 33.3 g/L NiSO$_4$, 11.1 g/L Pd(en)Cl$_2$, 16.70 g/L ammonium sulphate, 45ml/L of 35% ammonia and remainder water. The deposition potential was -1.1V with no pulse applied.

In another example, a sample has been fabricated with 20% Ni from a bath with 44.4 g/L NiSO4, 11.1 g/L Pd(en)Cl2, 16.70 g/L ammonium sulphate, 45ml/L of 35% ammonia and remainder water. The deposition potential was -1.2V with no applied pulse.

Figure 5 is a plan view of the test structure which was fabricated. The test chip has 12 sensors formed from 12 pairs of adjacent PdNi-Si Schottky barriers which can be seen distributed vertically in the figure. One of the 12 test devices is marked with reference numerals. The PdNi Schottky contacts 18 and 20 are labelled. The AI contact pads 22, 28 are labelled. Additionally end area portions 38, 40 of the respective contact pads 22, 28 are labelled, these end area portions being shaped to receive the respective contact probe leads 26, 28 (not illustrated). In the test structure, the Schottky contact pairs were fabricated to have the same area as each other, but with varying separations from each other (dimension M in Figures 3A & 3B) and varying overlaps with the aluminium contact pads (dimension L in Figures 3A & 3B), thus leaving different areas of the film exposed to gaseous hydrogen. $L_1=L_2=L$ in all cases.

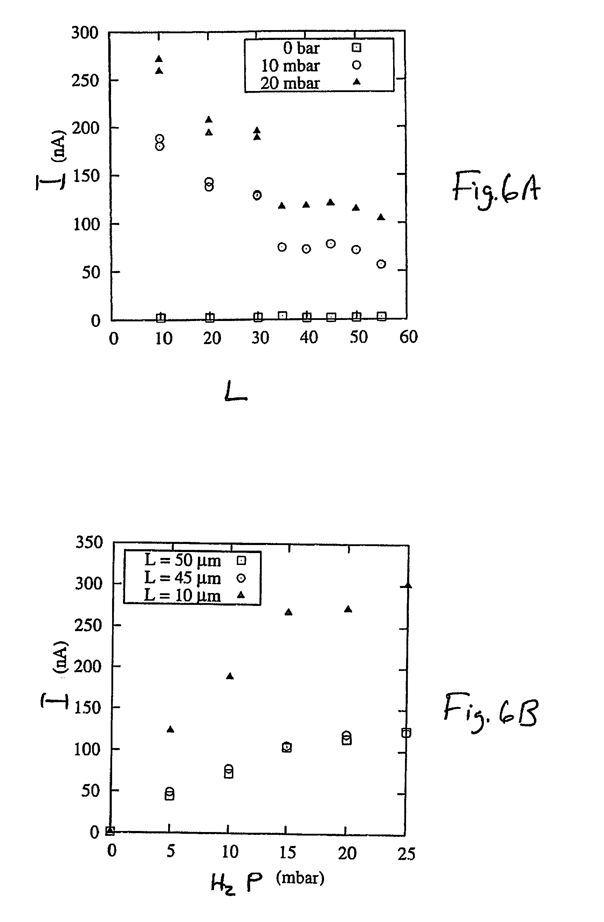

Figures 6A and 6B show hydrogen sensing results from an example sensing element with Schottky contacts made of $Pd_{0.71}Ni_{0.29}$

Figure 6A is a graph showing the dependence of device current I in nA on the exposed length L in $\mu$m of a sensing element with Schottky contacts made of $Pd_{0.7}Ni_{0.29}$.

Figure 6B is a graph showing the dependence of the current I in nA measured as a function of hydrogen pressure $H_2$ P in mbar for three example sensing elements with different values of the exposed length L.

To measure the response to gaseous hydrogen, the chip was placed inside the vacuum chamber of a Lakeshore EMTTP4 probe station and the chamber was evacuated to a pressure of approximately $1.5 \times 10^{-2}$ mbar. Nitrogen was introduced till a base pressure of 0.3 Bar and the sensor was allowed to settle for 5 minutes. A mixture of 5% hydrogen in nitrogen was then introduced in steps of 0.1 bar until the chamber pressure was a total of 1 bar. This is equivalent to introducing 5 mbar gaseous hydrogen as the hydrogen-nitrogen mixture has 5% hydrogen by volume. The back-to-back Schottky barrier response was measured by applying a bias of 1V and measuring the change in current with time at room temperature using an Agilent 4155C semiconductor parameter analyser. The sensor current increased rapidly on introduction of gaseous hydrogen and took a long time (40 minutes) to saturate. All the devices on the chip were measured at each pressure and the devices were allowed to rest 40 minutes before measurement so that they achieve their steady state currents.

Figure 6A shows the measured currents in the sensor device as a function of the exposed length of the PdNi film and for different pressures. It can be seen that the back-to-back Schottky barrier currents are very low in the absence of gaseous hydrogen and were measured to be between 1 - 2 nA and shows that the sensor draws ultra low power in the absence of hydrogen. Exposure of the sensing element to 10 mBar of hydrogen causes a large increase in the measured current of the device, with some samples showing increases greater by a factor of 100. Subsequent increases in hydrogen pressure cause further increases in the measured current, but the current saturates at higher hydrogen pressures.

Figure 6B shows the current saturation at higher hydrogen pressures. The current saturates at around 250-300 nA which is very low. The sensor therefore draws low power even in the presence of hydrogen. This low power consumption over the entire range of measured hydrogen pressures makes the sensor useful for systems with power constraints. The sensitivity of the sensor can be estimated by the slope of the initial linear part of the graph. For the three devices shown in Figure 6B, the highest sensitivity is found to be 17.27 nA/mbar and the lowest was 6.75 nA/mbar.

It is intuitive to expect that the change in current in the device will be positively correlated with the exposed length of the sensing element. However, Figure 6A shows an inverse relationship between the two. This can be explained by the fact that the devices are allowed to rest for 40 minutes so that they achieve their steady state currents. Using the value for the hydrogen diffusion constant as measured by Flanagan and Oates [14], it can be seen that this time is sufficient for hydrogen to diffuse through the entire PdNi film. The effect of the different exposed lengths is therefore negated.

In summary of the first embodiment, a hydrogen sensing device has been described which comprises: a semiconductor layer; first and second metal contacts made of a metal which has the property of disassociating hydrogen molecules and arranged adjacent to each other on the semiconductor layer, the first and second metal contacts having respective first and second interface surfaces that form first and second Schottky barriers with the semiconductor layer, and respective first and second sensing surfaces exposed to the atmosphere; and a sensing circuit operable to measure current that flows in a path from the first metal contact into the semiconductor and from the semiconductor to the second metal contact, the first and second metal contacts thereby forming back-to-back Schottky diodes in the sensing circuit, said current being sensitive to molecular hydrogen present at the sensing surfaces.

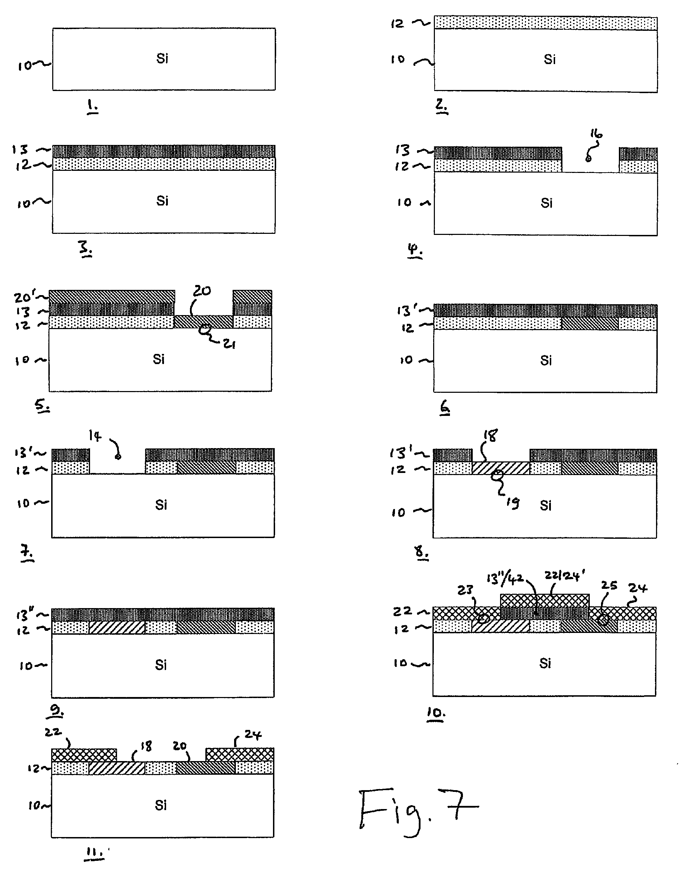

Figure 7 shows schematic cross-sections at various stages during fabrication of a device structure according to a second embodiment.

With reference to the introduction, it will be understood that the first embodiment described above with reference to Figures 1 to 6 relates to a device of the fourth type, namely a back-to-back Schottky structure with the hydrogen disassociating metal forming both contacts with the semiconductor. The second embodiment relates to a device of the third type, namely a device in which the contact pair is provided by a Schottky contact made of hydrogen disassociating metal and an Ohmic contact made of a different metal.

Because the fabrication in the second embodiment involves lift-off steps, some of the schematic cross-sections in Figure 7 show the photoresist layers which present at various stages, whereas these were omitted from the illustrations of the first embodiment shown in Figure 1.

In Stage 1, a semiconductor surface layer 10 is provided which may be the substrate itself as illustrated, or may be a separate semiconductor layer arranged on a substrate. In this example, a silicon substrate is provided in the form of an n-type 2-inch wafer of resistivity 0.5-1.5 Ωcm and crystallographic orientation <100>, which provides the semiconductor surface layer. In other words, there is no epitaxy involved. The wafer was cleaned by a 2 minute dip in fuming nitric acid (FNA) followed by rinsing with deionised (DI) water.

In Stage 2, an oxide layer 12 is grown on the substrate to act as an insulating layer. In the case of a Si surface, a $SiO_2$ layer can be grown thermally. In the specific example, a 50nm $SiO_2$, has been grown on the wafer.

In Stage 3, a resist 13 is spun onto the oxide 12.

In Stage 4, the oxide layer 12 is patterned using photolithography and the exposed oxide is etched by a 20:1 hydrofluoric acid (HF) etch to form a window 16 through the resist and oxide to the semiconductor surface.

In Stage 5, a metal is evaporated that is selected to provide an Ohmic contact to the underlying n-type silicon, such as AuSb alloy. The evaporation process deposits a blanket of the metal that fills not only the window area 16, but also the oxide layer 13. The metal film covering the oxide layer 13 is indicated with reference numeral 20'. At the interface 21 between the metal contact 20 and the semiconductor 10, an Ohmic junction is formed.

In Stage 6, the resist is removed to "lift off" the excess metal 20', and then a fresh photoresist layer 13' is spun onto the oxide.

In Stage 7, the oxide layer 12 is patterned using photolithography and the exposed oxide is etched by a 20:1 hydrofluoric acid (HF) etch to form a window 14 through the oxide to the semiconductor surface.

In Stage 8, a metal which has hydrogen catalysing properties is deposited using an electrodeposition process onto the semiconductor surface in the areas defined by the window 14 to form a metal contact 18. At the interface 19 between the metal contact 18 and the semiconductor 10, a Schottky barrier is formed. In this example, the hydrogen catalysing metal is a nickel-palladium alloy. Other metals that disassociate hydrogen molecules can be used, such as pure Pd, Pt or a Pt alloy. For the electrodeposition, the entire wafer is immersed in an electrochemical bath containing a salt of the metal and the metal is electrodeposited on the exposed silicon surface to form Schottky diodes. The electrodeposition process is described separately below.

In Stage 9, the resist layer 13' is removed and then a fresh resist layer 13" is spun onto the oxide layer 12.

In Stage 10, the resist 13" is patterned using photolithography and the exposed oxide is etched to form a bridge 42 of the resist 13" which covers the oxide between the contacts 18 and 20 and adjacent portions of the contacts 18 and 20. A further metal is then evaporated to provide contact pad electrodes to facilitate attachment of probe leads or wires. As illustrated, contact pads 22, 24 are formed to contact the respective Schottky contacts 18, 20. The further metal is selected so as to form an Ohmic contact at the respective interfaces 23, 25 between the contact pads 22, 24 and the metal contacts 18, 20. The further metal is aluminium in this example. The thickness of the aluminium contact pads is approximately 100 nm in this example.

In Stage 11, lift off is performed to remove the excess further metal 22/24' and also to expose a portion of the upper surface of the contacts 18 and 20. In fact, it is only necessary to expose a portion of the Schottky contact 18, since Ohmic contact 20 has no hydrogen activity. In other words, the resist bridge 42 need not cover any part of the contact 20.

In a variant of the second embodiment, electrodeposition is also used to form the Ohmic contact 20.

It will be understood that further details of the second embodiment may be understood with reference to the description of the first embodiment, i.e. Figures 2 to 6 and supporting description.

In summary of the second embodiment, a hydrogen sensing device has been described which comprises: a semiconductor layer; first and second metal contacts arranged adjacent to each other on the semiconductor layer, wherein the first metal contact is made of PdNi alloy which has the property of disassociating hydrogen molecules and forms a Schottky barrier with the semiconductor layer, and has a sensing surface exposed to the atmosphere; the second metal contact is made of a material that forms an Ohmic contact with the semiconductor layer; and a sensing circuit operable to measure current that flows in a path from the first metal contact into the semiconductor via the Schottky barrier and from the semiconductor to the second metal contact via the Ohmic contact, said current being sensitive to molecular hydrogen present at the sensing surface.

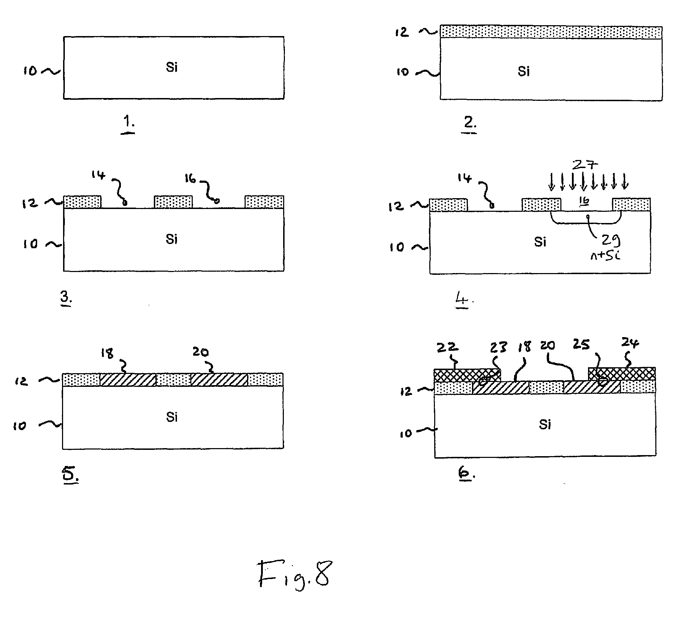

Figure 8 shows schematic cross-sections at five stages during fabrication of a device structure according to a third embodiment. The embodiment will be described with reference to a specific example, but the structure is generic to a range of materials combinations and other variations as specified elsewhere in this document.

In Stage 1, a semiconductor surface layer 10 is provided which may be the substrate itself as illustrated, or may be a separate semiconductor layer arranged on a substrate. In this example, a silicon substrate is provided in the form of an n-type 2-inch wafer of resistivity 0.5-1.5 Ωcm and crystallographic orientation <100>, which provides the semiconductor surface layer. In other words, there is no epitaxy involved. The wafer was cleaned by a 2 minute dip in fuming nitric acid (FNA) followed by rinsing with deionised (DI) water.

In Stage 2, an oxide layer 12 is grown on the substrate to act as an insulating layer. In the case of a Si surface, a $SiO_2$ layer can be grown thermally. In the specific example, a 50nm $SiO_2$, has been grown on the wafer.

In Stage 3, the oxide layer 12 is patterned using photolithography and the exposed oxide is etched by a 20:1 hydrofluoric acid (HF) etch to form adjacent windows 14, 16 through the oxide to the semiconductor surface.

In Stage 4, an ion implantation with ions 27 is then carried out through window 16 with a dopant to create degenerately doped n-type Si region 29 under the window area 16.

In Stage 5, a metal which has hydrogen catalysing properties is deposited using an electrodeposition process onto the semiconductor surface in the areas defined by the windows 14, 16 to form first and second metal contacts 18 and 20. This is the same process as described for the first embodiment. The window 14 will give PdNi/n-Si (>0.1 Ωcm) which will give a good Schottky barrier across interface 19, whereas the other window 16 with the ion implantation region 29 will give PdNi/n-Si (<<0.01 Ωcm) which will give an Ohmic contact across interface 21.

In Stage 6, a second photolithography step is carried out to define patterns of a further metal. As illustrated, contact pads 22, 24 are formed to contact the respective Schottky and Ohmic contacts 18, 20, while at the same time leaving a substantial area portion of the upper surface of at least the Schottky contact 18 exposed to adsorb hydrogen. The further metal is selected so as to form an Ohmic contact at the respective interfaces 23, 25 between the contact pads 22, 24 and the metal contacts 18, 20. The further metal is aluminium in this example and is deposited by evaporation and lift-off. The thickness of the aluminium contact pads is approximately 100 nm in this example.

In a variant, Stage 3 could be limited to forming only the second window. An additional lithography step would then be needed between Stages 4 and 5 to form the first window.

While certain embodiments have been described, these embodiments have been presented by way of example only, and are not intended to limit the scope of the inventions. Indeed, the novel methods, computers and computer program products described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope and spirit of the inventions.

## REFERENCES

[1] 1. Lundström, S. Shivaraman, C. Svensson, and L. Lundkvist. A Hydrogen sensitive MOS field effect transistor. Applied Physics Letters, 26(2), pages 55-57 (1975)

[2] R. C. Hughes and W. K. Schubert. Thin films of Pd/Ni alloys for detection of high Hydrogen concentrations. J. Appl. Phys., 71(1), pages 542-544 (1992)

[3] R. C. Hughes, W. K. Schubert, and R. J. Buss. Solid-state hydrogen sensors using Palladium-Nickel alloys, pages Effect of alloy composition on sensor response. Journal of The Electrochemical Society, 142(1), pages 249-254 (1995)

[4] US 6,935,158

[5] Jun-Rui Huang, Wei-Chou Hsu, Huey-Ing Chen, and Wen-Chau Liu. Comparative study of Hydrogen sensing characteristics of a Pd/GaN Schottky diode in air and Nitrogen atmospheres. Sensors and Actuators B: Chemical, volume 123(2), pages 1040-1048 (2007)

[6] US 5,367,283

[7] US 6,293,137

[8] US 6,160,278

[9] J. Schalwig, G. Müller, U. Karrer, M. Eickhoff, O. Ambacher, M. Stutzmann, L. Görgens, and G. Dollinger. Hydrogen response mechanism of Pt-GaN Schottky diodes. Applied Physics Letters, 80(7), pages 1222-1224 (2002)

[10] C. Weichsel, O. Pagni, and A. W. R. Leitch. Electrical and hydrogen sensing characteristics of Pd/ZnO Schottky diodes grown on GaAs. Semiconductor Science and Technology, 20(8), pages 840-843 (2005)

[11] Wang, Wang, Xiao, Feng, Wang, Wang, Yang, Wang, Wang, Ran, Hu and Li,.Hydrogen sensors based on Pt-AlGaN/GaN back-to-back Schottky diodes. Phys. Stat. Solidi volume 5, pages 2979-2981 (2008)

[12] M. Schlesinger, M. Paunovic, Modern Electroplating, 4th Edition, John Wiley and Sons, 2000.

[13] S.-E. Nam, K.-H. Lee. A study on the Palladium/Nickel composite membrane by vacuum electrodeposition, Journal of Membrane Science volume 170(1), pages 91-99 (2000)

[14] T. B. Flanagan, W. A. Oates. The Palladium-Hydrogen system, Annual Review of Materials Science volume 21(1), pages 269-304 (1991)

[15] Garrido, Gómez and Vallés. Simultaneous electrodeposition and detection of platinum on silicon surfaces, J Electro. Chem. volume 441, pages 147-151 (1998)

**Claims**

1. A method of manufacturing a hydrogen sensor comprising:

    (a) providing a substrate terminating in a layer of semiconductor;
    (b) forming an insulating layer on the semiconductor layer;
    (c) selectively removing part of the insulating layer to create a first window to expose a first area of the underlying semiconductor; and then
    (d) depositing by electrodeposition onto the first exposed area of the semiconductor a first contact made of a metal that disassociates hydrogen molecules and that forms a Schottky barrier with the semiconductor.

2. The method of claim 1, wherein the metal is PdNi alloy.

3. The method of claim 1, wherein the metal is Pd.

4. The method of claim 1, wherein the metal is Pt.

5. The method of any one of claims 1 to 4, wherein the semiconductor is Si.

6. The method of any one of claims 1 to 5, wherein the selective removal step additionally creates a second window to expose a second area of the underlying semiconductor adjacent to the first area, and wherein the deposition step additionally forms a second Schottky barrier contact of said metal on the second exposed area of the semiconductor, thereby to provide a sensing circuit of two back-to-back diodes interconnected by a current path through the semiconductor layer by simultaneous electrodeposition of the first and second contacts.

7. The method of any one of claims 1 to 5, further comprising the steps of:

    (e) selectively removing a further part of the insulating layer to create a second window that exposes a second area of the underlying semiconductor adjacent to the first window; and then
    (f) depositing a second contact on the second exposed area of the semiconductor layer, wherein the second contact is made of a further metal which forms an Ohmic contact with the semiconductor,

    thereby to provide a sensing circuit of a Schottky contact which in use acts as a diode in reverse bias and an Ohmic contact, the Schottky contact and the Ohmic contact being interconnected by a current path through the semiconductor layer.

8. The method of any one of claims 1 to 5, wherein the selective removal step or a further selective removal step creates a second window to expose a second area of the underlying semiconductor adjacent to the first area, and wherein a region of the semiconductor underlying the second area is doped through the second window, and wherein the deposition step additionally forms a second contact of said metal on the second exposed area of the semiconductor, said metal forming an Ohmic contact at the second area by virtue of the doped region,

thereby to provide a sensing circuit of a Schottky contact and an Ohmic contact interconnected by a current path through the semiconductor layer by simultaneous electrodeposition of the first and second contacts.

9. The method of claims 6, 7 or 8, wherein a fabrication step is performed after deposition of the first and second contacts to deposit first and second contact pads in Ohmic contact with the first and second contacts.

10. The method of any preceding claim, further comprising forming a further insulator layer in the first area, and optionally also the second area, between the metal and semiconductor.

11. A hydrogen sensor comprising:

(i) a semiconductor layer;
(ii) first and second metal contacts made of a PdNi alloy which has the property of disassociating hydrogen molecules and arranged adjacent to each other on the semiconductor layer, the first and second metal contacts having respective first and second interface surfaces that form first and second Schottky barriers with the semiconductor layer, and respective first and second sensing surfaces exposed to the atmosphere; and
(iii) a sensing circuit operable to measure current that flows in a path from the first metal contact into the semiconductor and from the semiconductor to the second metal contact, the first and second metal contacts thereby forming back-to-back Schottky diodes in the sensing circuit, said current being sensitive to hydrogen present at the sensing surfaces.

12. A hydrogen sensor comprising:

(i) a semiconductor layer;
(ii) first and second metal contacts arranged adjacent to each other on the semiconductor layer, wherein the first metal contact is made of PdNi alloy which has the property of disassociating hydrogen molecules and forms a Schottky barrier with the semiconductor layer, and has a sensing surface exposed to the atmosphere, and wherein the second metal contact forms an Ohmic contact with the semiconductor layer; and
(iii) a sensing circuit operable to measure current that flows in a path from the first metal contact into the semiconductor via the Schottky barrier and from the semiconductor to the second metal contact via the Ohmic contact, said current being sensitive to hydrogen present at the sensing surface.

13. The hydrogen sensor of claim 12, wherein the second metal is different from the first metal and forms an Ohmic contact with the semiconductor.

14. The hydrogen sensor of claim 12, wherein the second metal is the same as the first metal and forms an Ohmic contact with the semiconductor by virtue of local doping of the semiconductor at its interface with the second metal.

10 ~ | Si

<u>1.</u>

12 ~
10 ~ | Si

<u>2.</u>

14      16
12 ~
10 ~ | Si

<u>3.</u>

18      20
12 ~
10 ~ | Si

<u>4.</u>

22  23  18  20  25    24
12 ~
10 ~ | Si

<u>5.</u>

Fig. 1

Fig. 2

Fig. 3A

Fig. 3B

Fig. 4

Fig. 5

Fig.6A

Fig.6B

Fig. 7

Fig. 8

| | Europäisches Patentamt European Patent Office Office européen des brevets | **EUROPEAN SEARCH REPORT** | Application Number EP 10 29 0469 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KALTENPOTH G ET AL: "MULTIMODE DETECTION OF HYDROGEN GAS USING PALLADIUM-COVERED SILICON MU-CHANNELS", ANALYTICAL CHEMISTRY, AMERICAN CHEMICAL SOCIETY, US, vol. 75, no. 18, 15 September 2003 (2003-09-15), pages 4756-4765, XP001175750, ISSN: 0003-2700, DOI: DOI:10.1021/AC034507E | 1-3,5, 11,12 | INV. G01N27/12 G01N33/00 |
| Y | * page 4756, right-hand column, line 1 - page 4757, right-hand column, line 6 * * Scheme 1 * * page 4762, right-hand column, paragraph 2 - page 4764, right-hand column, paragraph 3; figure 11 * ----- | 4 | |
| Y | KIM SUKU ET AL: "Sensitivity of Pt/ZnO Schottky diode characteristics to hydrogen", APPLIED PHYSICS LETTERS, AIP, AMERICAN INSTITUTE OF PHYSICS, MELVILLE, NY, US, vol. 84, no. 10, 8 March 2004 (2004-03-08) , pages 1698-1700, XP012060716, ISSN: 0003-6951, DOI: DOI:10.1063/1.1664012 * abstract * ----- | 4 | TECHNICAL FIELDS SEARCHED (IPC) G01N |
| A | US 2007/069313 A1 (ENQUIST FREDRIK [SE]) 29 March 2007 (2007-03-29) * paragraph [0051] * * paragraph [0086] * ----- | 1-14 | |
| A | US 2002/182767 A1 (CHEN HUEY-ING [TW] ET AL) 5 December 2002 (2002-12-05) * the whole document * ----- | 1-14 | |

The present search report has been drawn up for all claims

| Place of search | Date of completion of the search | Examiner |
|---|---|---|
| The Hague | 28 January 2011 | Joyce, David |

EPO FORM 1503 03.82 (P04C01)

## ANNEX TO THE EUROPEAN SEARCH REPORT
## ON EUROPEAN PATENT APPLICATION NO.

EP 10 29 0469

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2011

| Patent document cited in search report | Publication date | Patent family member(s) | Publication date |
|---|---|---|---|
| US 2007069313 A1 | 29-03-2007 | NONE | |
| US 2002182767 A1 | 05-12-2002 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6935158 B
- US 5367283 A
- US 6293137 B
- US 6160278 A

**Non-patent literature cited in the description**

- **1. LUNDSTRÖM ; S. SHIVARAMAN ; C. SVENSSON ; L. LUNDKVIST.** A Hydrogen sensitive MOS field effect transistor. *Applied Physics Letters,* 1975, vol. 26 (2), 55-57
- **R. C. HUGHES ; W. K. SCHUBERT.** Thin films of Pd/Ni alloys for detection of high Hydrogen concentrations. *J. Appl. Phys.,* 1992, vol. 71 (1), 542-544
- **R. C. HUGHES ; W. K. SCHUBERT ; R. J. BUSS.** Solid-state hydrogen sensors using Palladium-Nickel alloys, pages Effect of alloy composition on sensor response. *Journal of The Electrochemical Society,* 1995, vol. 142 (1), 249-254
- **JUN-RUI HUANG ; WEI-CHOU HSU ; HUEY-ING CHEN ; WEN-CHAU LIU.** Comparative study of Hydrogen sensing characteristics of a Pd/GaN Schottky diode in air and Nitrogen atmospheres. *Sensors and Actuators B: Chemical,* 2007, vol. 123 (2), 1040-1048
- **J. SCHALWIG ; G. MÜLLER ; U. KARRER ; M. EICKHOFF ; O. AMBACHER ; M. STUTZMANN ; L. GÖRGENS ; G. DOLLINGER.** Hydrogen response mechanism of Pt-GaN Schottky diodes. *Applied Physics Letters,* 2002, vol. 80 (7), 1222-1224
- **C. WEICHSEL ; O. PAGNI ; A. W. R. LEITCH.** Electrical and hydrogen sensing characteristics of Pd/ZnO Schottky diodes grown on GaAs. *Semiconductor Science and Technology,* 2005, vol. 20 (8), 840-843
- **WANG ; WANG ; XIAO ; FENG ; WANG ; WANG ; YANG ; WANG ; WANG ; RAN.** Hydrogen sensors based on Pt-AIGaN/GaN back-to-back Schottky diodes. *Phys. Stat. Solidi,* 2008, vol. 5, 2979-2981
- **M. SCHLESINGER ; M. PAUNOVIC.** Modern Electroplating. John Wiley and Sons, 2000
- **S.-E. NAM ; K.-H. LEE.** A study on the Palladium/Nickel composite membrane by vacuum electrodeposition. *Journal of Membrane Science,* 2000, vol. 170 (1), 91-99
- **T. B. FLANAGAN ; W. A. OATES.** The Palladium-Hydrogen system. *Annual Review of Materials Science,* 1991, vol. 21 (1), 269-304
- **GARRIDO ; GÓMEZ ; VALLÉS.** Simultaneous electrodeposition and detection of platinum on silicon surfaces. *J Electro. Chem.,* 1998, vol. 441, 147-151